# EUROPEAN PATENT SPECIFICATION

(11) **EP 0 873 291 B1**
(45) Date of publication and mention of the grant of the patent: **16.01.2002**
(21) Application number: 95936310.2
(22) Date of filing: 06.10.1995
(51) Int. Cl.: C07C 15/073, C07C 15/085

(54) **PROCESS FOR MANUFACTURING ETHYLBENZENE OR CUMENE**
VERFAHREN ZUR HERSTELLUNG VON ETHYLBENZOL ODER VON CUMOL
PROCEDE DE FABRICATION D'ETHYLBENZENE OU DE CUMENE

(30) Priority: 06.10.1994 US 319212
(43) Date of publication of application: 28.10.1998
(73) Proprietor: ExxonMobil Chemical Patents Inc., Baytown, TX 77520-5200 (US)
(72) Inventor: HENDRIKSEN, Dan Eldon, Kingwood, TX 77345 (US); LATTNER, James Richardson, Seabrook, TX 77586 (US); ZBORAY, James Andrew, Houston, TX 77005 (US); SOLED, Stuart Leon, Pittstown, NJ 08867 (US)
(74) Representative: White, Nicholas John, Dr.
(86) International application number: US9513100
(87) International publication number: WO9611175

(56) References cited:
- EP-A- 0 402 202
- WO-A-93/20029
- US-A- 5 177 285

## Description

This invention relates to petroleum refining and petrochemistry, particularly to a process for manufacturing ethylbenzene from a composition containing benzene and another composition containing ethylene. Ethylbenzene is used commercially primarily as a raw material in the manufacture of styrene. This invention relates also to a process for manufacturing cumene from a composition containing benzene and another composition containing propylene. Cumene is used essentially as a raw material in the manufacture of phenol.

The known processes for the manufacture of ethylbenzene use the Friedel-Crafts reaction of alkylation of benzene by ethylene. Similarly, Friedel-Crafts reaction of alkylation of benzene by propylene is used to manufacture cumene.

The catalysts for this reaction are typically Bronsted or Lewis acids, including aluminum chloride, boron trifluoride deposited on alumina, or zeolites used in liquid or gas phase.

One of the difficulties encountered in using this reaction is that the ethylbenzene formed is more reactive than benzene with respect to ethylene, which leads to the production of diethylbenzenes, which are themselves more reactive than ethylbenzene, and therefore have a tendency to form triethylbenzenes.

To limit these polyalkylation reactions, the prior art teaches the use of a strong excess of benzene with respect to the ethylene at the entry of the alkylation reactors.

Thus, the benzene/ethylene molar ratio is generally between 2 and 2.5 for the processes using aluminum chloride, and the ratio may even reach a value between 8 and 16 for processes using zeolites in gas phase.

In spite of the use of an excess of benzene with respect to the ethylene to minimize the formation of polyethylbenzenes, such formation cannot be completely avoided. Thus, a typical alkylate composition obtained at the reactor outlet is given in the publication Petrochemical Processes, Edition Technip, Vol. I, p. 400, 1985:

| Percent by weight of total weight: | |
|---|---|
| Benzene | 38-40% |
| Ethylbenzene | 41-43% |
| Diethylbenzenes | 12-14% |
| Triethylbenzenes | 2-3% |
| Higher polyethylbenzenes having more than 3 ethyl groups | 3-4% |

In all known processes, the polyethylbenzenes, that is, the phenyl compounds which are substituted by at least two ethyl groups, are isolated before undergoing a transalkylation reaction by an excess of benzene according to:

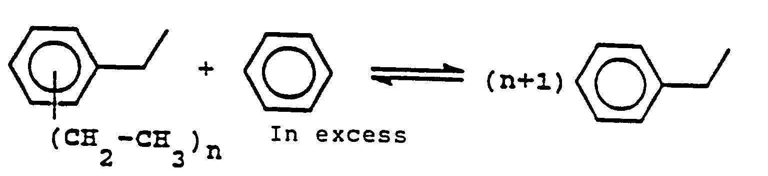

This reaction, catalyzed by the same catalysts as the alkylation reaction above, can be used in a reactor separate from the alkylation reactor, or in the alkylation reactor itself, which functions with an excess of benzene to encourage the preponderant formation of ethylbenzene.

A detailed description of these known techniques for the manufacture of ethylbenzene is included in Petrochemical Processes, mentioned above, or in the Encyclopedia of Chemical Technology, 3rd Edition, John Wiley and Sons, Vol. 21, p. 772 and following.

In all the known processes cited above, the benzene and the ethylene used in the alkylation reaction are each products of sufficient purity to lead to technical grade ethylbenzene, which can then be purified to the desired degree by distillation-rectification.

Petroleum refining technologies and the principle products resulting from them have also been described in Encyclopedia of Chemical Technology, 3rd Edition, Vol. 17, p. 183 and following.

EP 0402202 is directed towards producing an alkylbenzene from a mono olefin in the presence of a dealuminzed Y zeolite. An object of this invention is to improve the alkylation yield in mono-alkylbenzene by reducing the proportion of polyalkyl-benzenes.

Thus, certain heavy fractions obtained by distillation of petroleum are catalytically cracked in fluid catalytic cracking (FCC) to yield lighter products, which are put on the market as high-octane gasolines. This cracking also provides a certain proportion of so-called FCC gases, of which a light fraction comprises saturated hydrocarbons having two or fewer carbons and ethylene. This ethylene is present in a proportion by weight generally lower than 30% in this light fraction. The recovery of this ethylene by liquefaction followed by distillation is not profitable, and the use of this ethylene essentially diluted by methane and ethane has already been described, particularly in GB-1 013 268, US-A-3,131 ,230, US-A-3,200,164, and US-A-3,205,277.

In the alkylation processes of these patents, the benzene/ethylene ratio is greater than 2 and is generally greater than the ratio used in the known processes involving practically pure ethylene, so that the ethylene present in this light fraction can be exhausted (see also: Process Economics Program, Stanford Research Institute, Report no. 33, October 1986, p. 37).

The resulting alkylate is preponderant in benzene and includes a small quantity of ethyl benzene and polyethylbenzenes. This involves a significant cost for separating the ethylbenzene from this mixture. Thus, the light fraction of the FCC gases is not generally valued economically for its ethylene content.

The benzene used in the processes described above generally comes from sections out of the vapor cracking element of a petrochemical plant, or from sections coming from the reforming unit of a crude oil refinery.

The head fractions from a reformer may contain a significant proportion of benzene, and are, therefore, the source of a significant part of world benzene production.

However, when the reformer is small with respect to the refinery as a whole, or when it is fed by fractions that are too heavy to yield significant quantities of benzene, for economic reasons, the benzene is then left in the gasolines. However, considering the known toxicity of benzene, its presence in gasolines poses problems in terms of current or future regulations for reformulated gasolines.

It would be desirable to have a process to manufacture ethylbenzene from dilute feedstocks, rather than having to first recover both the pure benzene and the pure ethylene from dilute streams. For example, it would be desirable if a diluted ethylene stream, such as that from FCC gases, could be used as the ethylene feedstock, rather than requiring a pure ethylene stream as the feedstock to make ethylbenzene.

At the same time, it would be desirable to be able to economically remove the majority of the benzene from gasoline streams to meet environmental regulations, and also be able to use it as the benzene feedstock in the process to make ethylbenzene, rather than requiring a pure benzene stream as the feedstock.

An approach to the problems discussed above is disclosed in WO 93/20029. However, in most of the examples provided, aluminum chloride ethyl chloride catalyst systems are used for the alkylation and transalkylation reactions. These systems are known to have a number undesirable characteristics specifically due to the chloride catalyst systems. These catalyst systems are extremely corrosive to equipment, requiring ceramic lined vessels and expensive alloy materials of construction for most of the process equipment and creating many operational problems. Additionally, there is a significant environmental disposal problem associated with the use of aluminum chloride catalyst systems in benzene alkylation and transalkylation processes. The aluminum chloride catalyst operates in a once-through mode, and therefore the effluent from the process contains residual aluminum chloride and is often difficult to dispose of in an environmentally acceptable manner.

In one example in WO 93/20029, a small-pore mordenite with the aluminum removed, and a Si/AI ratio of 25, is used as the catalyst for the alkylation step, but not the transalkylation step. A large amount of mordenite relative to the benzene is required to effect the alkylation reaction, possibly because mordenite has been reported to rapidly lose its activity during the alkylation of benzene by ethylene.

Accordingly, it would be desirable to discover an alkylation catalyst system capable of functioning in the regime of impure ethylene and impure benzene described above and without the corrosion, environmental, and activity problems associated with the catalysts identified in the prior art as being efficient for this purpose.

### SUMMARY

This invention provides a process for manufacturing ethylbenzene from a first stream containing benzene and a second stream containing ethylene, comprising:
a) reacting said first stream with said second stream in an alkylation reaction of benzene by ethylene with a supported heteropolyacid or a Y zeolite catalyst to obtain a third stream consisting of an alkylate stream containing mono- and poly- ethylbenzenes in which the benzene ring to ethyl group molar ratio, including the unreacted benzene, in the alkylate stream, is equal to or less than 1.5;
b) subjecting this third stream consisting of an alkylate stream to at least one dlstlllation/rectification to obtain a fourth stream which is a distillate containing poly-ethylbenzenes;
c) transalkylating this fourth stream consisting of the distillate with a fifth stream consisting of benzene with a supported heteropolyacid or a Y zeolite catalyst to obtain a sixth stream consisting of a transalkylate rich in monoethylbenzene; and
d) subjecting this sixth stream consisting of a transalkylate to at least one distillation-rectification to obtain a seventh stream consisting of ethylbenzene.

In another embodiment, the heteropolyacid comprises phosphotungstic acid on a silica support. In yet another embodiment the Y zeolite catalyst is in the acid form. The poly-ethylbenzene component includes di-, tri-, tetra- penta-, and/or hexa-ethylbenzene.

In yet another embodiment, the benzene/ethylene molar ratio in the alkylation reaction is less than 1.

In another embodiment the first stream is a light reformate.

In another embodiment, the second stream also includes saturated hydrocarbons to the exclusion of unsaturated hydrocarbons other than ethylene. In yet another embodiment, the second stream is a distillation fraction of a fluid catalytic cracking gas including hydrocarbons with 2 or fewer carbons.

In another embodiment of the process, the fourth stream, which comprises the distillate of step b) also contains monoethylbenzene, and this fourth stream undergoes at least one distillation-rectification to obtain an eighth stream comprising a head fraction containing monoethylbenzene and a ninth stream comprising a tail fraction containing poly- ethylbenzenes.

In another embodiment of the process, the eighth stream comprising a head fraction containing monoethylbenzene is recycled to step a) to cause alkylation of this monoethylbenzene into poly-ethylbenzenes.

In another embodiment of the process, a tenth stream comprising a gasoline fraction consisting of saturated hydrocarbons and with a majority of the benzene removed is recovered by an additional distillation-rectification.

The above embodiments may be modified to produce cumene by substituting propylene for the ethylene.

### DETAILED DESCRIPTION

The production of ethylbenzene from dilute ethylene and dilute benzene is accomplished in three steps. First, the benzene feedstock stream, containing benzene, is alkylated with the dilute ethylene feedstock stream, containing ethylene, to form ethylbenzene along with polyethylbenzenes (PEB) which includes a mixture of di- and triethylbenzene that may also contain tetra-, penta-, and hexaethylbenzenes. Second, the product of the first step is distilled to remove the unreacted benzene and other unreacted material. Some of the ethylbenzene is recovered by distillation. In a third step, the polyethylbenzenes are transalkylated with excess pure benzene to form product ethylbenzene. The product ethylbenzene may be recovered by distillation.

The alkylation catalysts used in this invention are supported heteropolyacids, or Y zeolites used in liquid and / or gas phase.

The benzene feedstock stream may be pure benzene or diluted benzene containing at least 10 wt % benzene. The stream preferably contains at least 20 wt.% benzene and most preferably at least 30 wt.% benzene.

Advantageously, the alkylation reaction is industrially feasible with the use of a benzene feedstock stream also containing saturated hydrocarbons diluting the benzene which is present.

Advantageously, the benzene feedstock stream containing diluted benzene in the saturated hydrocarbons is a light reformate coming from a crude oil refinery. The possibility of using these generally abundant light reformates and eliminating their benzene content makes available gasolines which best fulfill current regulations.

The ethylene feedstock stream may be a distillation fraction of the gas from fluid catalytic cracking, or from a steam cracker containing hydrocarbons having 2 or fewer carbons. The ethylene feedstock stream may be pure ethylene or diluted ethylene containing at least 10 wt % ethylene. The stream preferably contains at least 20 wt.% ethylene and most preferably at least 30 wt.% ethylene.

Advantageously, the ethylene feedstock stream contains ethylene, which is diluted in saturated hydrocarbons, with the exception of unsaturated hydrocarbons other than ethylene. Thus, the absence of propylene, for example, simplifies the composition of the alkylate obtained and allows an easy separation of its constituents, particularly by one or more distillation-rectification operations.

When the benzene feedstock stream is a light fraction of reformate available in a refinery, we then see the benefit of the process according to the invention, which makes it possible to process most of the ethylene contained in the FCC gases from the refinery with all the benzene available in the light reformate.

In the first step, the two feedstock streams are brought together, under effective operating conditions, which are well known in the art, for the alkylation of benzene by the ethylene.

It is preferable for the benzene/ethylene molar ratio to be between 0.3 and 1 so that practically all the benzene put into reaction will be reacted with a sufficiently rapid rate of the alkylation reaction of benzene by ethylene.

It is preferable for the distillation rectification operation(s) or step b) to be regulated so that said distillate of step b) no longer contains monoethylbenzene, which is obtained by at least one distillation-rectification to obtain a head fraction with monoethylbenzene and a tail fraction with diand tri-ethylbenzene and heavy elements.

Preferably, this head fraction containing mono-ethylbenzene is recycled in stage a) to bring about alkylation of this monoethylbenzene into di- and tri-ethylbenzene. In this way the alkylate coming from the alkylation reactor is richer in di- and tri-ethylbenzene than the alkylate obtained without recycling and the benzene contained in the benzene feedstock stream combines with a greater number of ethylene molecules; it is a better carrier of the ethyl group in the transalkylation of step c).

Since the benzene feedstock stream is a light reformate, it is advantageous to recover in step b), by another distillation-rectification operation, a gasoline fraction formed of saturated hydrocarbons with a majority of the benzene removed.

Transalkylation of step c) of the process according to the invention is performed according to known processes, however the proportion of material undergoing transalkylation is greater than in conventional processes. The feed rate of benzene used is then adjusted to conform to customary ratios of benzene/ethyl groups. This transalkylation can be implemented on the same site where the alkylation of stage a) is conducted, or at another site where sufficient quantities of benzene are available. In the latter case, the polyethylbenzenes based on di- and tri-ethylbenzene can be introduced into a classic alkylation unit at the entry point of the alkylation or the transalkylation reactor.

This process utilizing dilute benzene feedstock may also be applied to the manufacture of cumene, rather than ethylbenzene, by using a dilute propylene feedstock stream, rather than a dilute ethylene feedstock stream.

In addition to this description, the invention can be better understood with the following examples.

### EXAMPLES

### Example 1

This Example illustrates the process of the invention for production of ethylbenzene using a supported phosphotungstic acid catalyst.

### Alkylation Catalyst Preparation.

The supported phosphotungstic acid catalyst is prepared by the method of incipient wetness as follows, The silica power (Davison 62) is calcined at 600°C overnight, and the phosphotungstic acid is dried at 120°C overnight. The dried phosphotungstic acid (21.43 g) is dissolved in 75cm³ of water and this solution is added to the calcined silica powder (50g). The resulting material is first dried overnight at 120°C and then calcined at 300°C for three hours. This results in a 30% by weight loading of phosphotungstic acid on the silica. Before use, the catalyst was dried again at 200°C under vacuum overnight.

### First Step -Alkylation

In a 300 cm³ magnetically stirred autoclave were placed dry benzene (40.4 g). dry hexanes (59,0 g). and a catalyst composed of 30% by weight of phosphotungstic acid supported on silica powder (9.6 g) prepared as described above. The autoclave was sealed, purged with hydrogen to remove air, and then pressured with 345 kPa (50 psi) gauge of hydrogen. The autoclave was heated to 180°C with stirring and then an additional pressure of 105 kPa (15 psig) of ethylene was added to the autoclave from a reservoir through a pressure regulator. As ethylene was consumed in the reaction more ethylene was added from the reservoir to maintain the pressure in the autoclave. After 17 hours the ethylene supply was closed, the reactor was cooled and vented, and the contents were removed. The amount of ethylene consumed was not directly measured.

A second alkylation reaction was run in the same manner to duplicate the results and to make more product. The materials initially placed in the autoclave were dry benzene (41.0 g), dry hexanes (59.7 g), and 30% by weight of phosphotungstic acid supported on silica powder (9.7 g) prepared as described above. The reaction was run for 17.5 hours.

The catalyst was filtered from the product of each reaction, and the products were combined and analyzed by capillary column gas chromatography. The combined product was found to contain 21.4 g of benzene and 62.4 g of ethylbenzene and polyethylbenzenes with the following composition by weight:

| EB | di-EB | tri-EB | tetra-EB | penta-EB | hexa-EB |
|---|---|---|---|---|---|
| 34.3% | 23.3% | 13.8% | 6.8% | 8.3% | 13.4% |

Thus, 73.7% of the benzene was converted. The mixture of ethylbenzene and polyethylbenzenes has a molar ratio of ethyl groups to benzene rings (Et/Bz) of 2.2. The Et/Bz molar ratio in the entire product, including unreacted benzene, is 1.4. The only difference between the two ratios is the unreacted benzene. These molar ratios may also be expressed as Bz/Et of 0.45 and 0.7 respectively.

### Second Step -Distillation

Distillation is used to first separate the hexanes and unreacted benzene from the ethylbenzene and PEB product, and then to separate the ethylbenzene from the mixture of polyethylbenzenes before transalkylation of this PEB mixture to make more ethylbenzene.

In this example, distillation of the combined products removed all of the hexanes and benzene, and some of the ethylbenzene product (10.4g). A small amount of diethylbenzene was unintentionally removed (1.6g). After the distillation the remaining material (62.4g) was dissolved in a small amount of benzene (28.3g) and analyzed by capillary column gas chromatography. There was found 51.0g of a mixture of ethylbenzene and polyethylbenzene with the following composition by weight:

| EB | di-EB | tri-EB | tetra-EB | penta-EB | hexa-EB |
|---|---|---|---|---|---|
| 9.7% | 24.9% | 19.7% | 10.7% | 13.3% 2 | 21.7% |

### Third Step -Transalkylation

To a 300 cm³ magnetically stirred autoclave was added 22.0 g of the mixture of ethylbenzene and polyethylbenzenes remaining after distillation (with added benzene), additional dry benzene (94.0 g), and a catalyst composed of 30% by weight of phosphotungstic acid supported on silica powder (10.0 g) prepared as described above. Thus, the Et/Bz molar ratio in the reactants was 0.17 or Bz/Et = 5.9. The autoclave was sealed, purged with nitrogen to remove air, and then pressured with 345 kPa (50psi) gauge of nitrogen. The autoclave was heated to 180°C with stirring. After 17.5 hours the reactor was cooled and vented, and the contents were removed and analyzed by capillary column gas chromatography. A yield of 15.5g of ethylbenzene was found. Thus, transalkylation of the entire mixture of ethylbenzene and polyethylbenzenes remaining after distillation would yield 63.9 g of ethylbenzene (EB), which combined with the EB recovered by distillation in the second step is 74.3 g EB. Those skilled in the art will recognize that both the remaining polyethylbenzenes in the transalkylation product and the remaining excess benzene may be recovered and recycled to another transalkylation reaction to yield additional ethylbenzene.

### Example 2

This Example illustrates the production of ethylbenzene using a Y zeolite catalyst in the acid form (LZY-84. UOP) for both reaction steps.

### Alkylation Catalyst Preparation

This catalyst was received as 1.6 mm (1/16 inch) extrudates. Before use it was crushed to a powder and dried under vacuum at 200°C overnight. All other procedures except for the catalyst preparation are the same as in Example 1.

### First Step -Alkylation

In a 300 cm³ magnetically stirred autoclave were placed dry benzene (42.2 g). dry hexanes (62.4 g), and a Y zeolite catalyst (LZY-84, UOP, 8.8 g) prepared as described above. The autoclave was sealed, purged with hydrogen to remove air, and then pressured with 345 kPa (50 psig) guage of hydrogen. The autoclave was heated to 180°C with stirring and then an additional pressure of 105 kPa (15 psi) of ethylene was added to the autoclave from a reservoir through a pressure regulator. As ethylene was consumed in the reaction more ethylene was added from the reservoir to maintain the pressure in the autoclave. After 17.5 hours the ethylene supply was closed, the reactor was cooled and vented, and the contents were removed.

A second alkylation reaction was run in the same manner. The materials initially placed in the autoclave were dry benzene (43.8 g), dry hexanes (60.5 g), and Y zeolite catalyst (LZY-84, UOP, 7.8 g) prepared as described above. The reaction was run for 17.5 hours.

The catalyst was filtered from the product of each reaction, and the products were analyzed by capillary column gas chromatography. The combined product was found to contain 20.5 g of benzene and 68.8 g of ethylbenzene and polyethylbenzenes with the following composition by weight:

| EB | di-EB | tri-EB | tetra-EB | penta-EB | hexa-EB |
|---|---|---|---|---|---|
| 50.1% | 23.0% | 12.5% | 6.6% | 5.8% | 2.1% |

Thus, 76% of the benzene was converted. The mixture of ethylbenzene and polyethylbenzenes has a molar ratio of ethyl groups to benzene rings (Et//Bz) of 1.7 or a Bz/Et ratio of 0.59. The Et//Bz molar ratio in the entire product (including unreacted benzene) is 1.2, which may be expressed as Bz/Et of 0.83.

### Second Step -Distillation

Distillation of the combined products removed all of the hexanes and benzene, and some of the ethylbenzene product (19.0 g). A small amount of diethylbenzene was unintentionally removed (1.7 g). After the distillation the remaining material (66.6 g) was rinsed out of the distillation pot with a small amount of benzene (4.9 g) and analyzed by capillary column gas chromatography. There was found 48.1 g of a mixture of ethylbenzene and polyethylbenzene with the following composition by weight:

| EB | di-EB | tri-EB | tetra-EB | penta-EB | hexa-EB |
|---|---|---|---|---|---|
| 29.9% | 29.9% | 18.2% | 10.0% | 8.8% | 3.2% |

### Third Step -Transalkylation

To a 300 cm³ magnetically stirred autoclave was added 26.1 g of the mixture of ethylbenzene and polyethylbenzenes remaining after distillation (with added benzene), additional dry benzene (109.0 g), and a Y zeolite catalyst (LZY -84, UOP, 10.2 g) prepared as described above. Thus, the Et//Bz molar ratio in the reactants was 0.18 or expressed as a Bz/Et molar ratio of 5.6. The autoclave was sealed, purged with nitrogen to remove air, and then pressured with 345 kPa. (50 psi) gauge of nitrogen. The autoclave was heated to 180°C with stirring. After 20.7 hours the reactor was cooled and vented, and the contents were removed and analyzed by capillary column gas chromatography. A yield of 33.0 g of ethylbenzene was found. Thus, transalkylation of the entire mixture of ethylbenzene and polyethylbenzenes remaining after distillation would yield 90.6 g of EB, which combined with the EB recovered by distillation in the second step, is 109.6 g EB. Those skilled in the art will recognize that both the remaining polyethylbenzenes in the transalkylation product and the remaining excess benzene may be recovered and recycled to another transalkylation reaction to yield additional ethylbenzene.

## Claims

1. A process for manufacturing (i) ethylbenzene or (ii) cumene from a first stream containing benzene and (i) a second stream containing ethylene, or (ii) a second stream containing propylene comprising:
a) alkylating the first stream with the second stream to obtain (i) an alkylate stream containing mono- and poly-ethylbenzenes or (ii) an alkylate stream containing monoand poly- isopropylbenzenes
b) subjecting the alkylate stream to at least one distillation/rectification to obtain a distillate stream containing (i) poly- ethylbenzenes or (ii) polyisopropylbenzenes;
c) transalkylating the distillate stream with a benzene stream to obtain a transalkylate stream rich in (i) monoethylbenzene or (ii) cumene; and
d) subjecting the transalkylate stream to at least one distillation-rectification to obtain (i) ethylbenzene or (ii) cumene;
**characterized in that** in step (a) the first and second streams are reacted in amounts such that in the alkylate stream the molar ratio of benzene rings (including those of unreacted benzene) (i) to ethyl groups or (ii) to propyl groups is equal to or less than 1.5: and that the benzene to ethylene or propylene molar ratio in the alkylation reaction is less than 1; and **in that** the alkylation step (a) and the transalkylation step (c) are carried out in the presence of a supported heteropolyacid catalyst or a Y zeolite catalyst.

2. A process according to Claim 1 wherein the catalyst used in steps (a) and (c) for ethylbenzene manufacture is a supported heteropolyacid catalyst.

3. A process according to Claim 1 wherein the catalyst used in steps (a) and (c) for ethylbenzene manufacture is a Y zeolite.

4. A process according to Claim 1 wherein the catalyst used in steps (a) and (c) for ethylbenzene manufacture are supported heteropolyacid catalyst and a Y zeolite respectively.

5. A process according to Claim 1 wherein the catalyst used in steps (a) and (c) for ethylbenzene manufacture is a Y zeolite and supported heteropolyacid respectively.

6. A process according to any of claims 1, 2, 4, and 5, wherein the supported heteropolyacid catalyst comprises a supported phosphotungstic acid catalyst.

7. A process according to claim 6, wherein the catalyst comprises phosphotungstic acid on a silica support.

8. A process according to claim 7, wherein the catalyst comprises approximately 30% by weight phosphotungstic acid on a silica support.

9. A process according to any of claims 1, 3, 4, and 5, wherein the Y zeolite catalyst is in the acid form.

10. A process according to any of the preceding claims, wherein the poly-ethylbenzene component includes di-, tri-, tetra- penta-, and/or hexa-ethylbenzene.

11. A process according to any of the preceding claims, wherein the first stream also contains saturated hydrocarbons.

12. A process according to claim 11, wherein the first stream is a light reformate.

13. A process according to any of the preceding claims, wherein the second stream also includes saturated hydrocarbons to the exclusion of unsaturated hydrocarbons other than (i) ethylene or (ii) propylene.

14. A process for ethylbenzene manufacture according to any of the preceding claims, wherein the second stream is a distillation fraction of a fluid catalytic cracking gas including hydrocarbons with 2 or fewer carbons.

15. A process according to any of the preceding claims, wherein the distillate stream from step b) also contains (i) monoethylbenzene or (ii) monoisopropylbenzene, and is subjected to an additional step comprising at least one distillation-rectification to obtain a head fraction containing (i) monoethylbenzene or (ii) monoisopropylbenzene, and a tail fraction containing (i) poly-ethyl benzenes or (ii) polyisopropylbenzenes, which tail fraction is passed to step (c).

16. A process according to claim 15, wherein the head fraction containing (i) monoethylbenzene or (ii) monoisopropylbenzene, is recycled to step a) for alkylation of (i) this monoethylbenzene into poly-ethylbenzenes, or (ii) this monoisopropylbenzene into polyisopropylbenzenes.

17. A process according to any of the preceding claims, wherein a stream comprising a gasoline fraction consisting of saturated hydrocarbons and with the majority of the benzene removed is recovered by an additional dlstillation-rectification.

18. A process according to claim 1 for manufacturing cumene from a first stream containing benzene and a second stream containing propylene, comprising:
a) alkylating the first benzene stream with the second propylene stream to obtain an alkylate stream containing mono- and poly- isopropylbenzenes, the first and second streams being reacted in amounts such that in the alkylate stream the molar ratio of benzene rings (including those of unreacted benzene) to propyl groups is equal to or less than 1.5: and that the benzene to propylene molar ratio in the alkylation reaction is less than 1;
b) subjecting the alkylate stream to at least one distillation/rectification to obtain a distillate stream containing poly- isopropylbenzenes;
c) transalkylating this distillate stream with a benzene stream to obtain a transalkylate stream rich in cumene; and
d) subjecting the transalkylate stream to at least one distillation-rectification to obtain cumene.

## Patentansprüche

1. Verfahren zur Herstellung von (i) Ethylbenzol oder (ii) Cumol aus einem ersten Benzol enthaltenden Strom und (i) einem zweiten Ethylen enthaltenden Strom oder (ii) einem zweiten Propylen enthaltenden Strom, bei dem
a) der erste Strom mit dem zweiten Strom alkyliert wird, um (i) einen Mono- und Polyethylbenzole enthaltenden Alkylatstrom zu erhalten, oder (ii) einen Mono- und Polyisopropylbenzole enthaltenden Alkylatstrom zu erhalten,
b) der Alkylatstrom mindestens einer Destillation/Rektifikation unterworfen wird, um einen (i) Polyethylbenzole oder (ii) Polyisopropylbenzole enthaltenden Destillatstrom zu erhalten,
c) der Destillatstrom mit einem Benzolstrom transalkyliert wird, um einen an (i) Monoethylbenzol oder (ii) Cumol reichen Transalkylatstrom zu erhalten, und
d) der Transalkylatstrom mindestens einer Destillation/ Rektifikation unterworfen wird, um (i) Ethylbenzol oder (ii) Cumol zu erhalten,
**dadurch gekennzeichnet, dass** in Stufe (a) die ersten und zweiten Ströme in solchen Mengen umgesetzt werden, dass das Molverhältnis von Benzolringen (einschließlich derer des nicht umgesetzten Benzols) zu (i) Ethylgruppen oder (ii) Propylgruppen in dem Alkylatstrom kleiner oder gleich 1,5 ist, und das Molverhältnis von Benzol zu Ethylen oder Propylen in der Alkylierungsreaktion kleiner als 1 ist, und die Alkylierungsstufe (a) und die Transalkylierungsstufe (c) in Gegenwart eines trägergestützten Heteropolysäurekatalysators oder eines Zeolith-Y-Katalysators durchgeführt werden.

2. Verfahren nach Anspruch 1, bei dem der in den Stufen (a) und (c) zur Ethylbenzolherstellung verwendete Katalysator trägergestützter Heteropolysäurekatalysator ist.

3. Verfahren nach Anspruch 1, bei dem der in den Stufen (a) und (c) zur Ethylbenzolherstellung verwendete Katalysator ein Y-Zeolith ist.

4. Verfahren nach Anspruch 1, bei dem der in den Stufen (a) und (c) zur Ethylbenzolherstellung verwendete Katalysator trägergestützter Heteropolysäurekatalysator beziehungsweise ein Y-Zeolith ist.

5. Verfahren nach Anspruch 1, bei dem der in den Stufen (a) und (c) zur Ethylbenzolherstellung verwendete Katalysator ein Y-Zeolith beziehungsweise trägergestützter Heteropolysäurekatalysator ist.

6. Verfahren nach einem der Ansprüche 1, 2, 4 und 5, bei dem der trägergestützte Heteropolysäurekatalysator trägergestützten Phosphowolframsäurekatalysator enthält.

7. Verfahren nach Anspruch 6, bei dem der Katalysator Phosphowolframsäure auf einem Siliciumdioxidträger enthält.

8. Verfahren nach Anspruch 7, bei dem der Katalysator ungefähr 30 Gew.% Phosphowolframsäure auf einem Siliciumdioxidträger enthält.

9. Verfahren nach einem der Ansprüche 1, 3, 4 und 5, bei dem der Y-Zeolith-Katalysator in der Säureform vorliegt.

10. Verfahren nach einem der vorhergehenden Ansprüche, bei dem die Polyethylbenzolkomponente Di-, Tri-, Tetra-, Pentaund/oder Hexaethylbenzol enthält.

11. Verfahren nach einem der vorhergehenden Ansprüche, bei dem der erste Strom auch gesättigte Kohlenwasserstoffe enthält.

12. Verfahren nach Anspruch 11, bei dem der erste Strom ein leichtes Reformat ist.

13. Verfahren nach einem der vorhergehenden Ansprüche, bei dem der zweite Strom auch gesättigte Kohlenwasserstoffe enthält, wobei von (i) Ethylen oder (ii) Propylen verschiedene ungesättigte Kohlenwasserstoffe ausgeschlossen sind.

14. Verfahren zur Herstellung von Ethylbenzol nach einem der vorhergehenden Ansprüche, bei dem der zweite Strom eine Destillationsfraktion eines katalytischen Wirbelschichtcracker-Gases ist, das Kohlenwasserstoffe mit 2 oder weniger Kohlenstoffatomen enthält.

15. Verfahren nach einem der vorhergehenden Ansprüche, bei dem der Destillatstrom aus Stufe b) auch (i) Monoethylbenzol oder (ii) Monoisopropylbenzol enthält und einer zusätzlichen Stufe unterworfen wird, die mindestens eine Destillation/ Rektifikation umfasst, um eine (i) Monoethylbenzol oder (ii) Monoisopropylbenzol enthaltende Kopffraktion und eine. (i) Polyethylbenzole oder (ii) Polyisopropylbenzole enthaltende Endfraktion zu erhalten, wobei die Endfraktion an Stufe (c) weitergeleitet wird.

16. Verfahren nach Anspruch 15, bei dem die (i) Monoethylbenzol oder (ii) Monoisopropylbenzol enthaltende Kopffraktion zu Stufe a) zurückgeführt wird, um (i) dieses Monoethylbenzol zu Polyethylbenzolen oder (ii) dieses Monoisopropylbenzol zu Polyisopropylbenzolen zu alkylieren.

17. Verfahren nach einem der vorhergehenden Ansprüche, bei dem ein Strom, der eine Benzinfraktion enthält, die aus gesättigten Kohlenwasserstoffen besteht und deren überwiegender Anteil an Benzol entfernt worden ist, durch eine zusätzliche Destillation/Rektifikation gewonnen wird.

18. Verfahren nach Anspruch 1 zur Herstellung von Cumol aus einem ersten Benzol enthaltenden Strom und einem zweiten Propylen enthaltenden Strom, bei dem
a) der erste Benzolstrom mit dem zweiten Propylenstrom alkyliert wird, um einen Mono- und Polyisopropylbenzole enthaltenden Alkylatstrom zu erhalten, wobei der erste und der zweite Strom in solchen Mengen umgesetzt werden, dass das Molverhältnis von Benzolringen (einschließlich derer des nicht umgesetzten Benzols) zu Propylgruppen in dem Alkylatstrom kleiner oder gleich 1,5 ist, und das Molverhältnis von Benzol zu Propylen in der Alkylierungsreaktion kleiner als 1 ist,
b) der Alkylatstrom mindestens einer Destillation/Rektifikation unterworfen wird, um einen Polyisopropylbenzole enthaltenden Destillatstrom zu erhalten,
c) dieser Destillatstrom mit einem Benzolstrom transalkyliert wird, um einen an Cumol reichen Transalkylatstrom zu erhalten, und
d) der Transalkylatstrom mindestens einer Destillation/ Rektifikation unterworfen wird, um Cumol zu erhalten.

## Revendications

1. Procédé pour la production (i) d'éthylbenzène ou (ii) de cumène à partir d'un premier courant contenant du benzène et (i) un second courant contenant de l'éthylène, ou (ii) un second courant contenant du propylène, comprenant les étapes consistant :
a) à alkyler le premier courant avec le second courant pour obtenir (i) un courant de produits d'alkylation contenant des mono- et polyéthylbenzènes, ou (ii) un courant de produits d'alkylation contenant des mono- et polyisopropylbenzènes,
b) à soumettre le courant de produits d'alkylation à au moins une distillation/rectification pour obtenir un courant de distillat contenant (i) des polyéthylbenzènes ou (ii) des polyisopropylbenzènes ;
c) à transalkyler le courant de distillat avec un courant de benzène pour obtenir un courant de produits de transalkylation riche en (i) mono-éthylbenzène ou (ii) cumène ;
d) à soumettre le courant de produits de transalkylation à au moins une distillation-rectification pour obtenir (i) de l'éthylbenzène ou (ii) du cumène ;
**caractérisé en ce que**, dans l'étape (a), les premier et second courants sont amenés à réagir en des quantités telles que, dans le courant de produits d'alkylation, le rapport molaire des noyaux benzéniques (y compris ceux du benzène n'ayant pas réagi) (i) aux groupes éthyle ou (ii) aux groupes propyle soit égal ou inférieur à 1,5 ; et que le rapport molaire du benzène à l'éthylène ou au propylène dans la réaction d'alkylation soit inférieur à 1 ; et **en ce que** l'étape d'alkylation (a) et l'étape de transalkylation (c) sont mises en oeuvre en présence d'un catalyseur consistant en un hétéropolyacide fixé sur un support ou d'un catalyseur consistant en zéolite Y.

2. Procédé suivant la revendication 1, dans lequel le catalyseur utilisé dans les étapes (a) et (c) pour la production d'éthylbenzène est un catalyseur consistant en un hétéropolyacide fixé sur un support.

3. Procédé suivant la revendication 1, dans lequel le catalyseur utilisé dans les étapes (a) et (c) pour la production d'éthylbenzène est une zéolite Y.

4. Procédé suivant la revendication 1, dans lequel le catalyseur utilisé dans les étapes (a) et (c) pour la production d'éthylbenzène consiste, respectivement, en un catalyseur du type hétéropolyacide fixé sur un support et une zéolite Y.

5. Procédé suivant la revendication 1, dans lequel le catalyseur utilisé dans les étapes (a) et (c) pour la production d'éthylbenzène consiste, respectivement, en une zéolite Y et un hétéropolyacide fixé sur un support.

6. Procédé suivant l'une quelconque des revendications 1, 2, 4 et 5, dans lequel le catalyseur consistant en un hétéropolyacide fixé sur un support comprend un catalyseur du type acide phosphotungstique fixé sur un support.

7. Procédé suivant la revendication 6, dans lequel le catalyseur comprend de l'acide phosphotungstique sur un support consistant en silice.

8. Procédé suivant la revendication 7, dans lequel le catalyseur comprend approximativement 30 % en poids d'acide phosphotungstique sur un support consistant en silice.

9. Procédé suivant l'une quelconque des revendications 1, 3, 4 et 5, dans lequel le catalyseur consistant en zéolite Y est sous forme acide.

10. Procédé suivant l'une quelconque des revendications précédentes, dans lequel le constituant polyéthylbenzène comprend les di-, tri-, tétra-, penta-et/ou hexa-éthylbenzènes.

11. Procédé suivant l'une quelconque des revendications précédentes, dans lequel le premier courant contient également des hydrocarbures saturés.

12. Procédé suivant la revendication 11, dans lequel le premier courant est un reformat léger.

13. Procédé suivant l'une quelconque des revendications précédentes, dans lequel le second courant comprend également des hydrocarbures saturés à l'exclusion d'hydrocarbures insaturés autres que (i) l'éthylène ou (ii) le propylène.

14. Procédé pour la production d'éthylbenzène suivant l'une quelconque des revendications précédentes, dans lequel le second courant est une fraction de distillation d'un gaz de craquage catalytique fluide comprenant des hydrocarbures ayant deux ou moins de deux atomes de carbone.

15. Procédé suivant l'une quelconque des revendications précédentes, dans lequel le courant de distillat de l'étape b) contient également (i) du mono-éthylbenzène ou (ii) du mono-isopropylbenzène, et est soumis à une étape supplémentaire comprenant au moins une distillation-rectification pour obtenir une fraction de tête contenant (i) du mono-éthylbenzène ou (ii) du mono-isopropylbenzène, et une fraction de queue contenant (i) des polyéthylbenzènes ou (ii) des polyisopropylbenzènes, fraction de queue qui est passée à l'étape (c).

16. Procédé suivant la revendication 15, dans lequel la fraction de tête contenant (i) du mono-éthylbenzène et (ii) du mono-isopropylbenzène est recyclée à l'étape a) pour l'alkylation (i) de ce mono-éthylbenzène en polyéthylbenzènes, ou (ii) de ce mono-isopropylbenzène en polyisopropylbenzènes.

17. Procédé suivant l'une quelconque des revendications précédentes, dans lequel un courant comprenant une fraction d'essence consistant en hydrocarbures saturés, avec la plus grande partie du benzène éliminée, est recueilli par une distillation-rectification supplémentaire.

18. Procédé suivant la revendication 1 pour la production de cumène à partir d'un premier courant contenant du benzène et d'un second courant comprenant du propylène, comprenant les étapes consistants :
a) à alkyler le premier courant de benzène avec le second courant de propylène pour obtenir un courant de produits d'alkylation contenant des mono- et polyisopropyl-benzènes, les premier et second courants étant amenés à réagir en des quantités telles que, dans le courant de produits d'alkylation, le rapport molaire des noyaux benzéniques (y compris ceux du benzène n'ayant pas réagi) aux groupes propyle soit égal ou inférieur à 1,5 ; et que le rapport molaire du benzène au propylène dans la réaction d'alkylation soit inférieur à 1 ;
b) à soumettre le courant de produits d'alkylation à au moins une distillation/rectification pour obtenir un courant de distillat contenant des polyisopropylbenzènes ;
c) à transalkyler ce courant de distillat avec un courant de benzène pour obtenir un courant de produits de transalkylation riche en cumène ; et
d) à soumettre le courant de produits de transalkylation à au moins une distillation-rectification pour obtenir du cumène.
